Europäisches Patentamt

European Patent Office

Office européen des brevets

(19)

(11) Veröffentlichungsnummer: **0 232 265**
**B1**

(12) # EUROPÄISCHE PATENTSCHRIFT

(45) Veröffentlichungstag der Patentschrift:
29.11.89

(21) Anmeldenummer: **85904446.3**

(22) Anmeldetag: **04.09.85**

(86) Internationale Anmeldenummer:
**PCT/EP 85/00444**

(87) Internationale Veröffentlichungsnummer:
**WO 86/01892 (27.03.86** Gazette 86/7)

(51) Int. Cl.⁴: **G 01 N 21/66, G 03 G 17/00**

(54) **VERFAHREN UND VORRICHTUNG ZUM PRÜFEN VON FLÜSSIGEN ARZNEIMITTELN.**

(30) Priorität: **18.09.84 DE 3434154**

(43) Veröffentlichungstag der Anmeldung:
**19.08.87 Patentblatt 87/34**

(45) Bekanntmachung des Hinweises auf die Patenterteilung:
**29.11.89 Patentblatt 89/48**

(84) Benannte Vertragsstaaten:
**AT BE CH DE FR GB LI NL SE**

(56) Entgegenhaltungen:
**US-A- 2 920 201**

**Elektor, Bd.3, Nr.10, Oktober 1977, "Kirlian Photography", S. 10-05 - 10-08**
**Journal of Applied Physics, Bd. 44, Nr.7, Juli 1973, New York (US); D.Boyers et al.:"Corona discharge photography", S.3102-3112**

(73) Patentinhaber: **KNAPP, Dieter, Fahrenbacher Strasse 22, D-6149 Fürth/Odw. (DE)**

(72) Erfinder: **KNAPP, Dieter, Fahrenbacher Strasse 22, D-6149 Fürth/Odw. (DE)**

(74) Vertreter: **Katscher, Helmut, Dipl.-Ing., Bismarckstrasse 29, D-6100 Darmstadt (DE)**

## Beschreibung

Die Erfindung betrifft ein Verfahren und eine Vorrichtung zum Prüfen von flüssigen Arzneimitteln, insbesondere auch homöopathischen Präparaten.

Die Prüfung von Arzneimitteln kann herkömmlich durch chemische, chromatografische oder spektrometrische Verfahren sowie durch Untersuchungen an lebenden Organismen erfolgen. Daneben besteht die Möglichkeit der einfachen Prüfung mit den Sinnesorganen auf Trübung, Geruchsveränderung u. dgl.

Allen herkömmlichen Verfahren ist gemeinsam, daß sie entweder sehr ungenau oder nur wenig aussagekräftig sind. Deshalb muß man sich in den meisten Fällen darauf verlassen, daß eine Arzneimittellösung unter Beachtung gewisser Aufbewahrungsvorschriften, insbesondere hinsichtlich Temperatur und Lichteinwirkung, für eine bestimmte Zeitspanne brauchbar bleibt. Diese Zeitspanne, innerhalb deren ein Arzneimittel eingesetzt werden darf, muß vorsorglich verhältnismäßig kurz bemessen werden, um eine ausreichende Sicherheit zu erhalten. Wenn eine Schädigung des Arzneimittels ungewöhnlich früh eingetreten ist, beispielsweise infolge von unbemerkt gebliebenen äußeren Einflüssen, oder wenn ein Arzneimittel schon bei oder kurz nach der Herstellung Veränderungen erfahren hat, so ist dies entweder nur mit sehr aufwendigen Untersuchungsmethoden, die in den meisten Fällen nur im Herstellungsbetrieb angewandt werden können, oder überhaupt nicht mehr feststellbar.

Aufgabe der Erfindung ist es daher, ein Verfahren zum Prüfen von flüssigen Arzneimitteln zu schaffen, das sehr einfach durchgeführt werden kann und bei sehr unterschiedlichen Arzneimitteln anwendbar ist und das es gestattet, schon geringfügige Abweichungen von Vergleichsproben deutlich erkennbar zu machen, so daß sowohl bei frisch hergestellten Arzneimitteln als auch bei länger gelagerten Arzneimitteln eine rasche und umfassende Prüfung möglich ist, die auch solche Veränderungen erkennen läßt, die mit herkömmlichen Prüfverfahren nicht oder nur unzureichend oder nur mit sehr großem Verfahrensaufwand feststellbar sind.

Diese Aufgabe wird erfindungsgemäß dadurch gelöst, daß in einer dünnen, im Querschnitt angenähert keilförmigen Schicht des flüssigen Arzneimittels eine elektrische Koronaentladung ausgelöst wird, deren Bild optisch aufgezeichnet und ausgewertet wird.

Es hat sich überraschend gezeigt, daß im Bild einer solchen Koronaentladung schon geringfügige Änderungen des Arzneimittels, beispielsweise hinsichtlich einer Lösungskonzentration oder eines Alterungsvorganges, sehr deutlich erkennbar sind. Das Verfahren läßt außerdem solche Veränderungen, die auf die Wirkung des Arzneimittels Einfluß haben, mit anderen chemischen oder physikalischen Verfahren aber kaum erfaßbar sind, deutlich in Erscheinung treten, so daß biologische Prüfverfahren, wie Versuche an Tieren oder Zellkulturen, entbehrlich werden.

Es hat sich als zweckmäßig sowohl hinsichtlich der Versuchsdurchführung als auch hinsichtlich der Auswertbarkeit und Vergleichbarkeit der dabei erhaltenen Bilder ergeben, das Prüfverfahren so durchzuführen, daß die Koronaentladung zwischen einer konvex gewölbten Elektrodenfläche und einer Oberfläche eines Dielektrikums erfolgt. Der im Querschnitt keilförmige Spalt erstreckt sich dabei ringförmig um den Scheitelpunkt der gewölbten Elektrodenfläche.

Die optische Aufzeichnung erfolgt zweckmäßigerweise auf fotografischem Wege; daneben ist aber auch eine elektronische Bildspeicherung und eine elektronische Bildabtastung möglich, beispielsweise mittels eines Laserstrahls.

Gemäß einer vorteilhaften Ausgestaltung des Erfindungsgedankens ist vorgesehen, daß das flüssige Arzneimittel auf die Oberfläche einer fotografischen Schicht unmittelbar oder unter Zwischenlage eines Glas-Objektträgers aufgebracht wird und daß eine konvex gewölbte Elektrode auf die fotografische Schicht bzw. den Glas-Objektträger mit vorgegebener Kraft gedrückt wird. Dabei wird in der fotografischen Schicht, die auf einem fotografischen Film oder einer fotografischen Platte aufgebracht sein kann, eine besonders scharfe Aufzeichnung der Koronaentladungserscheinungen erzielt, insbesondere wenn sich das Arzneimittel auf der fotografischen Schicht befindet.

Die Erfindung betrifft außerdem eine Vorrichtung zur Durchführung des Verfahrens. Eine solche Vorrichtung ist bekannt aus «Corona discharge photography», Journal of Applied Physics, Band 44, Nr. 7, Juli 1973, New York (US), Seiten 3102-3112. Sie weist eine mit einem Hochfrequenzgenerator verbundene Flachelektrode, die an der Rückseite einer Isolierplatte angeordnet ist, sowie einen fotografischen Schichtträger auf, der zwischen der Isolierplatte und einer zweiten Elektrode angeordnet ist.

Die erfindungsgemäße Vorrichtung ist dadurch gekennzeichnet, daß die zweite Elektrode eine mit einer konvex gewölbten Kontaktfläche versehene bewegliche Elektrode ist, die gegen den fotografischen Schichtträger auf der der Fachelektrode abgewandten Seite der Isolierplatte drückbar ist. Diese Vorrichtung ist von verhältnismäßig einfachem Aufbau und ermöglicht eine rasche Prüfung, so daß auch mehrere Proben in kurzen Zeitabständen untersucht werden können. Weitere vorteilhafte Ausgestaltungen der Erfindung sind Gegenstand weiterer Unteransprüche.

Die Erfindung wird nachfolgend an Ausführungsbeispielen näher erläutert, die in der Zeichnung dargestellt sind. Es zeigt:

Fig. 1 in vereinfachter Darstellungsweise eine mit einem Hochfrequenzgenerator ausgerüstete Vorrichtung zur Untersuchung von flüssigen Arzneimitteln,

Fig. 2 in einer vergrößerten Darstellung entsprechend der Fig. 1 den konvex gewölbten Endabschnitt der mit dem Arzneimittel benetzten Elektrode, die auf die fotografische Schicht aufgesetzt ist, und

Fig. 3 in einer Darstellung ähnlich der Fig. 1 eine abgewandelte Ausführungsform der Vorrichtung.

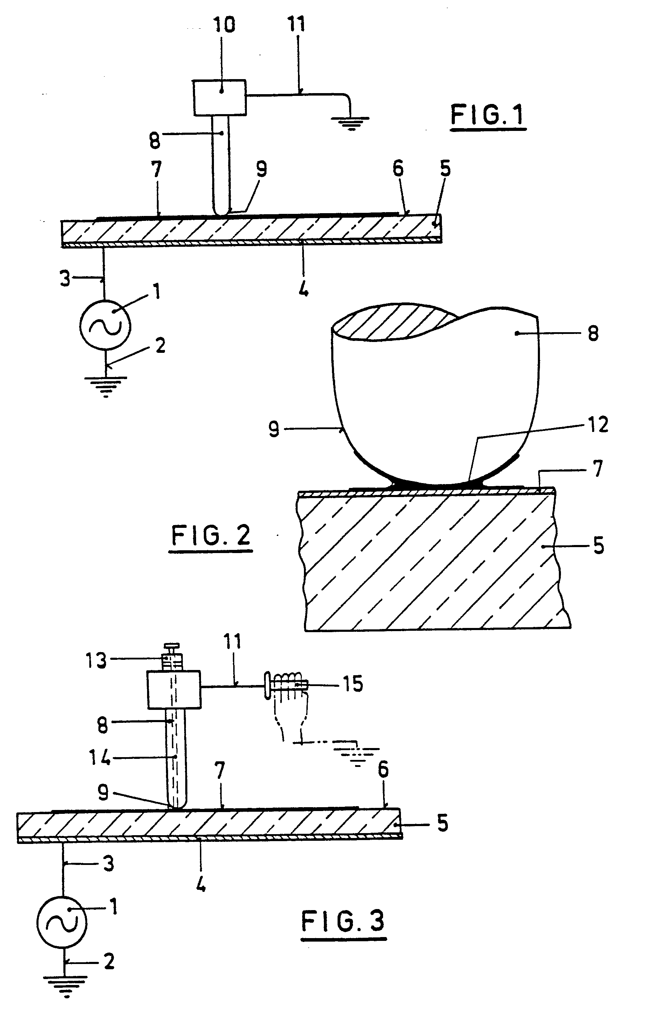

Die in Fig. 1 gezeigte Prüfvorrichtung weist einen Hochfrequenzgenerator 1 auf, dessen einer Anschluß 2 geerdet ist und dessen anderer Anschluß 3 mit einer Flachelektrode 4 verbunden ist, die an der Unterseite einer Glasscheibe 5 aufgebracht ist. Die Flachelektrode 4 kann beispielsweise als aufgedampfte Metallschicht ausgeführt sein.

Auf die Oberseite 6 der Glasscheibe 5 wird für jeden Prüfvorgang als fotografischer Schichtträger ein fotografischer Film 7 gelegt. Der Prüfvorgang erfolgt in einer Dunkelkammer.

Eine bewegliche Elektrode 8, beispielsweise ein Metallstab aus Messing oder einem anderen gut leitfähigen Material ist zylinderförmig ausgeführt und weist am unteren Ende eine konvex gewölbte Elektrodenfläche 9 auf, beim Ausführungsbeispiel einer Halbkugelfläche mit dem Radius der zylindrischen Elektrode 8.

Die Elektrode 8 trägt an ihrem oberen Ende ein Gewicht 10, das die Anpreßkraft der gewölbten Elektrodenfläche 9 an den fotografischen Film 7 bestimmt. Die Elektrode 8 ist über eine Leitung 11 geerdet (Fig. 1).

Von dem zu untersuchenden flüssigen Arzneimittel wird eine Probe 12 in den Bereich zwischen der gewölbten Fläche 9 und dem Film 7 gebracht. Da die Elektrode 8 mit ihrem Scheitel die Oberfläche des Films 7 berührt, füllt die Probe 12 einen im Querschnitt im wesentlichen keilförmigen Ringraum aus und benetzt die gewölbte Elektrodenfläche 9 und die Oberfläche des Films 7. Die zu untersuchende flüssige Probe 12 kann in sehr einfacher Weise dadurch aufgebracht werden, daß die Elektrode 8 zunächst in einen Behälter getaucht wird, der das zu untersuchende flüssige Arzneimittel enthält. Das Arzneimittel benetzt den eingetauchten Teil der Elektrode 8 und gelangt auch auf die Oberfläche des Films 7, wenn die gewölbte Elektrodenfläche 9 auf den Film 7 gedrückt wird. Statt dessen kann die zu untersuchende flüssige Probe auch zunächst auf die Oberfläche des Films 7 gegeben werden, bevor die Elektrode 8 aufgesetzt wird. Es versteht sich, daß die Elektrodenfläche 9 vor der Durchführung des Versuches sorgfältig gereinigt wird, beispielsweise durch Abreiben mit Alkohol.

Statt dessen ist es auch möglich, die Elektrode 8 selbst mit einer in Fig. 3 nur andeutungsweise dargestellten Dosiervorrichtung 13 zu versehen, durch die eine vorgegebene Menge des zu untersuchenden flüssigen Arzneimittels durch eine zentrale, in der gewölbten Kontaktfläche 9 mündende Zuführbohrung 14 in den Bereich zwischen der Kontaktfläche 9 und dem Film 7 gebracht wird.

Mittels des Hochfrequenzgenerators 1, der beispielsweise mit einer Taktfrequenz von 1 Hz bis 10 kHz betrieben wird und eine Spannung von 5 - 50 kV erzeugt, wird im Bereich um die Berührungsstelle zwischen der Elektrode 8 und dem Film 7 eine elektrische Koronaentladung erzeugt, die sich auf dem Film 7 abbildet. Nur Arzneimittelproben, die in ihrer Zusammensetzung und in ihrer chemischen und biologischen Wirkung gleich sind, ergeben übereinstimmende Entladungsbilder. Deutlich erkennbare Abweichungen in den Entladungsbildern lassen auf Veränderungen der zu untersuchenden Stoffe schließen. Beispielsweise können alterungsbedingte Veränderungen festgestellt werden, die mit anderen Untersuchungsverfahren nicht oder nur mit großem Aufwand und nur wenig zuverlässig erfaßt werden können.

Abweichend von dem Ausführungsbeispiel nach Fig. 1, bei dem die Elektrode 8 über die Leitung 11 unmittelbar geerdet ist, ist beim Ausführungsbeispiel nach Fig. 3 die mit der Elektrode 8 verbundene Leitung 11 mit einem Handgriff 15 elektrisch leitend verbunden. Wenn eine Person diesen Handgriff 15 ergreift, erfolgt die Erdung der Elektrode 8 über diese Person. Die bei dieser Anordnung erzeugten Entladungsbilder lassen Rückschlüsse auf die individuelle Wirksamkeit des untersuchten Arzneimittels zu.

Abweichend von den dargestellten Ausführungsbeispielen kann die zu untersuchende flüssige Probe 12 auch auf einen Glas-Objektträger aufgebracht werden, der auf dem Film 7 liegt. Es hat sich jedoch gezeigt, daß die schärfsten und damit aussagekräftigsten Bilder erhalten werden, wenn die Probe 12 unmittelbar auf die Oberfläche des Films 7 aufgebracht wird.

**Patentansprüche**

1. Verfahren zum Prüfen von flüssigen Arzneimitteln, dadurch gekennzeichnet, daß in einer dünnen, im Querschnitt angenähert keilförmigen Schicht des flüssigen Arzneimittels eine elektrische Koronaentladung ausgelöst wird, deren Bild optisch aufgezeichnet und ausgewertet wird.

2. Verfahren nach Anspruch 1, dadurch gekennzeichnet, daß die Koronaentladung zwischen einer konvex gewölbten Elektrodenfläche und einer Oberfläche eines Dieelektrikums erfolgt.

3. Verfahren nach Anspruch 1, dadurch gekennzeichnet, daß die optische Aufzeichnung auf fotografischem Wege erfolgt.

4. Verfahren nach Anspruch 3, dadurch gekennzeichnet, daß das flüssige Arzneimittel auf die Oberfläche eines fotografischen Schichtträgers unmittelbar oder unter Zwischenlage eines Glas-Objektträgers aufgebracht wird und daß die konvex gewölbte Elektrode auf den fotografischen Schichtträger bzw. den Glas-Objektträger mit vorgegebener Kraft gedrückt wird.

5. Vorrichtung zur Durchführung des Verfahrens nach einem der Ansprüche 1 bis 4, bei der eine mit einem Hochfrequenzgenerator (1) verbundene Flachelektrode (4) an der Rückseite einer Isolierplatte (5) angeordnet ist und ein fotografischer Schichtträger (7) zwischen der Isolierplatte (5) und einer zweiten Elektrode (8) angeordnet ist, dadurch gekennzeichnet, daß die zweite Elektrode (8) eine mit einer konvex gewölbten Kontaktfläche (9) versehene bewegliche Elektrode (8) ist, die gegen den fotografischen Schichtträger (7) auf der der Flachelektrode (4) abgewandten Seite (6) der Isolierplatte (5) drückbar ist.

6. Vorrichtung nach Anspruch 5, dadurch gekennzeichnet, daß die bewegliche Elektrode (8) ein zylindrischer Metallstab mit halbkugelförmiger Kontaktfläche (9) ist.

7. Vorrichtung nach Anspruch 5, dadurch gekennzeichnet, daß die bewegliche Elektrode (8) eine in der Kontaktfläche (9) mündende Zuführbohrung (14) für das flüssige Arzneimittel aufweist.

8. Vorrichtung nach Anspruch 5, dadurch gekennzeichnet, daß die bewegliche Elektrode (8) mit einer geerdeten Anschlußleitung (11) versehen ist.

9. Vorrichtung nach Anspruch 5, dadurch gekennzeichnet, daß die bewegliche Elektrode (8) elektrisch leitend mit einem Handgriff (15) verbunden ist.

## Claims

1. Process for testing liquid medicaments, characterised in that an electric corona discharge, the pattern of which is recorded optically and assessed, is triggered in a thin layer of the liquid medicament with an approximately wedge-shaped cross-section.

2. Process according to Claim 1, characterised in that the corona discharge occurs between a convexly domed electrode surface and a surface of a dielectric.

3. Process according to Claim 1, characterised in that the optical recording is carried out by photography.

4. Process according to Claim 3, characterised in that the liquid medicament is applied onto the surface of a photographic layer base directly or with an interlayer of a glass object carrier and that the convexly domed electrode is pressed at a predetermined force against the photographic layer base or the glass object carrier.

5. Device for carrying out the process according to one of Claims 1 to 4, in which a flat electrode (4) connected to a high-frequency generator (1) is arranged on the rear side of an insulating plate (5) and a photographic layer base (7) is arranged between the insulating plate (5) and a second electrode (8), characterised in that the second electrode (8) is a movable electrode (8) provided with a convexly domed contact surface (9), it being possible to press the electrode against the photographic layer base (7) on the side (6) of the insulating plate (5) turned away from the flat electrode (4).

6. Device according to Claim 5, characterised in that the movable electrode (8) is a cylindrical metal rod with a hemispherical contact surface (9).

7. Device according to Claim 5, characterised in that the movable electrode (8) has a feed hole (14) which debouches in the contact surface (9) for the liquid medicament.

8. Device according to Claim 5, characterised in that the movable electrode (8) is provided with an earthed connection cable (11).

9. Device according to Claim 5, characterised in that the movable electrode (8) is connected conductively to a handle (15).

## Revendications

1. Procédé pour la mise à l'épreuve de médicaments liquides, caractérisé en ce que, dans une couche mince (à section transversale à peu près cunéiforme) du médicament liquide, on déclenche une décharge électrique à effet de couronne dont l'image est enregistrée et évaluée optiquement.

2. Procédé selon la revendication 1, caractérisé en ce que la décharge à effet de couronne a lieu entre une surface d'électrode à bombement complexe et une surface d'un diélectrique.

3. Procédé selon la revendication 1, caractérisé en ce que l'enregistrement optique a lieu par voie photographique.

4. Procédé selon la revendication 3, caractérisé en ce que le médicament liquide est appliqué sur la surface d'un support de couche photographique directement ou en intercalant un porte-objet en verre, tandis que l'électrode à bombement convexe est pressée, avec une force prédéterminée, sur le support de couche photographique ou le porte-objet en verre.

5. Dispositif pour la réalisation du procédé selon une des revendications 1 à 4, dispositif dans lequel une électrode plate (4) raccordée à un générateur de haute fréquence (1) est disposée sur la face dorsale d'une plaque isolante (5), tandis qu'un support de couche photographique (7) est disposé entre la plaque isolante (5) et une deuxième électrode (8), caractérisé en ce que la deuxième électrode (8) est une électrode (8) mobile comportant une surface de contact (9) à bombement convexe, cette électrode pouvant être pressée, contre le support de couche photographique (7), sur la face (6) de la plaque isolante (5) qui est éloignée de l'électrode plate (4).

6. Dispositif selon la revendication 5, caractérisé en ce que l'électrode mobile (8) est une barre métallique cylindrique comportant une surface de contact hémisphérique (9).

7. Dispositif selon la revendication 5, caractérisé en ce que l'électrode mobile (8) comporte un passage d'alimentation (14) pour le médicament liquide, ce passage débouchant dans la surface de contact (9).

8. Dispositif selon la revendication 5, caractérisé en ce que l'électrode mobile (8) est pourvue d'une ligne de raccordement (11) mise à la terre.

9. Dispositif selon la revendication 5, caractérisé en ce que l'électrode mobile (8) est raccordée de manière électriquement conductrice à une poignée (15).

FIG.1

FIG. 2

FIG. 3